# FASCICULE DE BREVET EUROPEEN

(11) **EP 1 944 009 B1**
(45) Date de publication et mention de la délivrance du brevet: **06.11.2013**
(21) Numéro de dépôt: 08100319.6
(22) Date de dépôt: 10.01.2008
(51) Int. Cl.: A61K 8/38, A61K 8/19, A61K 8/23, A61K 8/37

(54) **Composition pour la décoloration des cheveux comprenant un ester liquide ramifié non volatile d'acide carboxylique à point de solidification inférieur à 4°c**
Haarbleichzusammensetzung enthaltend einen flüssigen nichtflüchtigen verzweigten Carbonsäureester mit einem Erstarrungspunkt unter 4°C
Hair bleaching composition comprising a non-volatile liquid branched ester of a carboxylic acid with a solidification point below 4°C

(30) Priorité: 15.01.2007 FR 0752677
(43) Date de publication de la demande: 16.07.2008
(73) Titulaire: L'Oréal, 75008 Paris (FR)
(72) Inventeur: Boche, Benoît, 92250 La Garenne Colombes (FR); Braida-Valerio, Damarys, 75012 Paris (FR); Nicolas-Morgantini, Luc, 60810 Rully (FR); Kravtchenko, Sylvain 168 Ji Nan Lu The Lakeville Block 9, Unit 2301, 200021 Shanghaï (CN)
(74) Mandataire: Prevel, Estelle Nicole

(56) Documents cités:
- EP-A- 1 258 237
- EP-A- 1 430 875
- EP-A- 1 462 088
- DE-A1-102005 003 412
- FR-A1- 2 842 100
- FR-A1- 2 842 101
- US-A- 5 342 829
- STAPLES, CHARLES A.; PETERSON, DENNIS R.; PARKERTON, THOMAS F.; ADAMS, WILLIAM J: "The environmental fate of phthalate esters : a literature review" CHEMOSPHERE, [Online] vol. 35, no. 4, août 1997 (1997-08), pages 667-670, XP002450499 ISSN: 0045-6535 Extrait de l'Internet: URL:http://dx.doi.org/10.1016/S0045-6535(9 7)00195-1> [extrait le 2007-09-12]

## Description

La présente invention a pour objet une composition pour la décoloration des fibres kératiniques, et en particulier des fibres kératiniques humaines telles que les cheveux, comprenant au moins un sel peroxygéné et au moins un ester liquide ramifié non volatile d'acide carboxylique dont le point de solidification est inférieur à 4 °C convenablement sélectionné.

La décoloration des fibres kératiniques humaines, et plus particulièrement des cheveux, se fait par oxydation du pigment "mélanine" aboutissant à la solubilisation et l'élimination partielle ou totale de ce pigment.

Pour décolorer les cheveux, on utilise des poudres décolorantes contenant un réactif peroxygéné tel que les persulfates, perborates et percarbonates, d'ammonium ou de métaux alcalins, que l'on associe au moment de l'emploi à une composition aqueuse de peroxyde d'hydrogène.

Les sels peroxygénés et le peroxyde d'hydrogène étant relativement stables en milieu acide, il est souvent nécessaire de les activer à pH basique pour obtenir une formation adéquate d'oxygène. Il est donc usuel d'ajouter aux poudres décolorantes, des composés alcalins tels que l'urée, les silicates et les phosphates de métaux alcalins ou alcalino-terreux, et en particulier les métasilicates de métaux alcalins, ou des agents précurseurs d'ammoniac comme les sels d'ammonium.

Les poudres décolorantes ont cependant tendance à former de la poussière durant leur manutention, leur transport et leur stockage.

Or, les produits qui les composent (persulfates, silicates alcalins) sont corrosifs, irritants pour les yeux, les voies respiratoires et les muqueuses.

Pour s'affranchir du problème de volatilité des poudres décolorantes, on a développé des pâtes qui comprennent lesdits agents pulvérulents (sels peroxygénés, agents alcalins, épaississants) dans un support liquide inerte organique. De telles compositions sont notamment décrites dans les demandes de brevets DE 38 14 356 et DE 197 23 538.

Mais les pâtes décolorantes basées sur cette technologie qui sont actuellement sur le marché ont une stabilité physico-chimique non satisfaisante, et ne permettent pas d'obtenir une décoloration suffisamment homogène et puissante. En outre, elles ne présentent pas un bel aspect.

Pour remédier au manque de stabilité des pâtes décolorantes, on a déjà eu recours à des associations d'agents épaississants convenablement choisis, tel qu'il est décrit dans les demandes de brevet EP 0 778 020 et EP 1 034 777.

Pour garantir une stabilité meilleure encore, on a également utilisé des cires qui épaississent le liquide inerte organique. Par cires, on entend notamment des produits dont le point de fusion est supérieur à 40 °C ou des esters d'acides gras hydrophobes à chaîne longue ou des produits de substitution de la cire d'abeille.

Mais dans ces conditions, pour être dispersée, voire solubilisée dans le liquide organique, la cire doit être fondue, ce qui implique de la chauffer au cours du procédé de fabrication.

Par ailleurs, de telles compositions à base de cires sont sensibles à la température et aux chocs thermiques, tant au cours de leur fabrication que de leur stockage. Les pâtes perdent alors leurs qualités d'usage.

Pour pallier ces inconvénients, il a été proposé, dans les demandes de brevet FR 2 842 099 et FR 2 842 100, d'utiliser l'association d'un liquide inerte organique et d'une silice pyrogénée à caractère hydrophile ou hydrophobe ou l'association d'un polydécène et d'un agent gélifiant choisi parmi les silices pyrogénées à caractère hydrophile ou hydrophobe et les copolymères di-, tri-, multi- ou radical blocs composés de segments de type styrène monomère et de segments de type monomère ou comonomère thermoplastique.

Cependant, les pâtes décolorantes actuellement commercialisées présentent encore l'inconvénient d'être peu résistantes au froid. On observe notamment des problèmes de synérèse, c'est-à-dire d'exsudation de la phase huileuse, lors de stockage à basses températures et lors de transport incluant des cycles de température.

Le but de la présente invention est de fournir une composition pour la décoloration des fibres kératiniques qui permette de résoudre le problème de volatilité des poudres sans présenter les inconvénients des compositions connues de l'art antérieur, en particulier une composition pour la décoloration des fibres kératiniques qui présente une bonne résistance aux basses températures, tout en permettant d'obtenir une décoloration puissante et homogène et en ne laissant les cheveux ni gras ni rêches.

Ce but est atteint avec la présente invention qui a pour objet une composition pour la décoloration des fibres kératiniques sous la forme d'une pâte anhydre comprenant au moins un sel peroxygéné et au moins un ester liquide ramifié non volatile d'acide carboxylique dont le point de solidification est inférieur à 4 °C choisi parmi les composés de structure (I) suivante :

R₁-CO-O-R₂ (I)

dans laquelle R₁ et R₂ désignent, indépendamment l'un de l'autre, une chaîne hydrocarbonée en C₁-C₃₀, de préférence en C₂-C₂₀, éventuellement interrompue par un ou plusieurs atomes d'oxygène et/ou par un ou plusieurs groupements carbonyle et éventuellement substituée par un ou plusieurs groupements hydroxy, R₁ étant ramifiée.

La présente invention a également pour objet un procédé de décoloration des fibres kératiniques mettant en oeuvre la composition conforme à l'invention, ainsi que des dispositifs à plusieurs compartiments pour la mise en oeuvre de ce procédé.

Un autre objet de la présente invention est l'utilisation d'un ester liquide ramifié non volatile d'acide carboxylique dont le point de solidification est inférieur à 4 °C tel que défini précédemment dans une composition pour la décoloration des fibres kératiniques sous la forme d'une pâte anhydre comprenant un sel peroxygéné.

La présente invention permet d'obtenir une composition pour la décoloration des fibres kératiniques qui présente une résistance aux basses températures améliorée, et en particulier qui permet d'éviter le problème de synérèse lors de stockage à basses températures et lors de transport incluant des cycles de température.

A moins d'une indication différente, les bornes des gammes de valeurs qui sont données dans le cadre de la présente invention sont incluses dans ces gammes.

Le ou les sels peroxygénés présents dans la composition conforme à l'invention peuvent par exemple être choisis parmi les persulfates, les perborates, les percarbonates, les peroxydes de métaux alcalins ou alcalino-terreux, et leurs mélanges. De préférence, on utilisera les persulfates et leurs mélanges, et plus préférentiellement les persulfates de sodium, de potassium et d'ammonium, et leurs mélanges.

La concentration en sels peroxygénés dans la composition conforme à l'invention est généralement comprise entre 10 et 70 % en poids, et de préférence entre 20 et 60 % en poids du poids total de la composition.

Par basse température, on entend au sens de la présente invention une température inférieure à 10 °C, et de préférence inférieure à 5 °C.

Par liquide, on entend au sens de la présente invention toute phase capable d'écoulement à température ambiante, généralement entre 15 °C et 40 °C, et à pression atmosphérique, sous l'action de son propre poids.

Par non volatile, on entend au sens de la présente invention un composé présentant une pression de vapeur inférieure ou égale à 5 mm de Hg à la température de 20 °C. De préférence, cette pression de vapeur est inférieure à 1 mm de Hg.

Par ester ramifié, on entend au sens de la présente invention un ester comportant dans la partie issue d'un acide et / ou dans la partie issue d'un alcool au moins une chaîne hydrocarbonée ramifiée comportant au moins trois atomes de carbone.

Le ou les esters liquides ramifiés non volatiles d'acides carboxyliques utiles dans le cadre de l'invention ont une température de solidification inférieure à 4 °C. Cette température de solidification peut être notamment déterminée par DSC (Differential Scanning Calorimetry). Comme appareil de DSC utilisable, on peut citer l'appareil PYRIS 1 de PERKIN ELMER.

Le ou les esters liquides ramifiés non volatiles d'acides carboxyliques dont le point de solidification est inférieur à 4 °C sont issus d'un acide ramifié.

Selon un mode de réalisation particulier de l'invention, le ou les esters liquides ramifiés non volatiles d'acides carboxyliques dont le point de solidification est inférieur à 4 °C comprennent au moins 8 atomes de carbone.

A titre d'esters utilisables dans la présente invention, on peut notamment citer, l'isononanoate d'octyle, l'isononanoate d'isononyle, l'isobutyrate d'isobutyle.

De préférence, R₁ et R₂ sont ramifiées. Le ou les esters liquides ramifiés non volatiles d'acides carboxyliques dont le point de solidification est inférieur à 4 °C sont alors issus d'un acide ramifié et d'un alcool ramifié.

Encore plus préférentiellement, on utilisera l'isononanoate d'isononyle

La concentration en esters liquides ramifiés non volatiles d'acides carboxyliques dont le point de solidification est inférieur à 4 °C dans la composition conforme à l'invention est généralement comprise entre 1 et 70 % en poids, et de préférence entre 5 et 60 % en poids, et encore plus préférentiellement entre 10 et 50 % en poids du poids total de la composition.

Selon un mode de réalisation particulier, la composition conforme à la présente invention comprend au moins un agent alcalin.

Le ou les agents alcalins peuvent par exemple être choisis parmi l'urée, les sels d'ammonium comme le chlorure d'ammonium, le sulfate d'ammonium, le phosphate d'ammonium ou le nitrate d'ammonium, les silicates, les phosphates ou les carbonates de métaux alcalins ou alcalino-terreux, tels que le lithium, le sodium, le potassium, le magnésium, le calcium, le baryum, et leurs mélanges. De préférence, le ou les agents alcalins sont choisis parmi le chlorure d'ammonium, les silicates, les carbonates, et leurs mélanges.

Lorsqu'ils sont présents dans la composition conforme à l'invention, la concentration en agents alcalins est généralement comprise entre 0,01 et 40 % en poids, et de préférence entre 0,1 et 30 % en poids du poids total de la composition.

Selon un mode de réalisation particulier, la composition conforme à l'invention comprend au moins un liquide inerte organique additionnel différent des esters liquides ramifiés non volatiles d'acides carboxyliques dont la température de solidification est inférieure à 4 °C.

Par liquide inerte organique, on entend au sens de la présente invention un liquide organique chimiquement inerte vis-à-vis du peroxyde d'hydrogène. Dans le cadre de l'invention, un liquide est inerte si la dégradation du peroxyde d'hydrogène en présence de ce liquide est inférieure à 25 % après 15 heures à 100 °C.

A titre d'exemple, on peut citer les polydécènes de formule C₁₀ₙH_{[(20n)+2]} dans laquelle n varie de 3 à 9 et de préférence de 3 à 7, les esters d'alcools gras ou d'acides gras différents des esters de l'invention, les esters ou di-esters de sucres et d'acides gras en C₁₂-C₂₄, les éthers cycliques ou les esters cycliques, les huiles de silicone, les huiles minérales ou les huiles végétales, ou leurs mélanges.

Les composés de formule C₁₀ₙ H_{[(20n)+2]} avec n variant de 3 à 9 répondent à l'appellation "polydécène" du Dictionnaire CTFA 7ème édition 1997 de la Cosmetic, Toiletry and Fragrance Association, USA, ainsi qu'à la même appellation I.N.C.I. aux USA et en Europe. Ce sont des produits d'hydrogénation des poly-1-décènes.

Parmi ces composés, on préfère selon l'invention ceux pour lesquels dans la formule, n varie de 3 à 7.

On peut citer à titre d'exemple le produit vendu sous la dénomination Silkflo® 366 NF Polydecene par la société Amoco Chemical, ceux vendus sous la dénomination Nexbase® 2002 FG, 2004 FG, 2006 FG et 2008 FG par la société Fortum.

En ce qui concerne les esters d'alcools gras ou d'acides gras différents des esters de l'invention, on peut citer à titre d'exemple :
- les esters de monoalcools inférieurs saturés linéaires ou ramifiés en C₃-C₆, avec des acides gras monofonctionnels en C₁₂-C₂₄, ces derniers pouvant être linéaires ou ramifiés, saturés ou insaturés et choisis notamment parmi les oléates, laurates, palmitates, myristates, béhénates, cocoates, stéarates, linoléates, linolénates, caprates, arachidonates, ou leurs mélanges comme notamment les oléo-palmitates, oléo-stéarates, palmito-stéarates.,. Parmi ces esters, on préfère plus particulièrement utiliser le palmitate d'isopropyle, le myristate d'isopropyle.
- les esters de monoalcools linéaires ou ramifiés en C₃-C₈, avec des acides gras bifonctionnels en C₈-C₂₄, ces derniers pouvant être linéaires ou ramifiés, saturés ou insaturés,
- les esters de monoalcools linéaires ou ramifiés en C₃-C₈, avec des acides gras bifonctionnels en C₈-C₂₄, ces derniers pouvant être linéaires ou ramifiés, saturés ou insaturés.
- l'ester d'un acide trifonctionnnel.

En ce qui concerne les esters et di-esters de sucres et d'acides gras en C₁₂-C₂₄, on entend par "sucre" des composés qui possèdent plusieurs fonctions alcool, avec ou sans fonction aldéhyde ou cétone, et qui comportent au moins 4 atomes de carbone. Ces sucres peuvent être des monosaccharides, des oligosaccharides ou des polysaccharides.

Comme sucres utilisables selon l'invention, on peut citer par exemple le sucrose (ou saccharose), le glucose, le galactose, le ribose, le fuctose, le maltose, le fructose, le mannose, l'arabinose, le xylose, le lactose, et leurs dérivés notamment alkylés, tels que les dérivés méthylés comme le méthylglucose.

Les esters de sucres et d'acides gras utilisables selon l'invention peuvent être choisis notamment dans le groupe comprenant les esters ou mélanges d'esters de sucres décrits ci-avant et d'acides gras en C₁₂-C₂₄, linéaires ou ramifiés, saturés ou insaturés.

Les esters peuvent être choisis parmi les mono-, di-, tri- et tétra-esters, les polyesters et leurs mélanges.

Ces esters peuvent être par exemple choisis parmi les oléates, laurates, palmitates, myristates, béhénates, cocoates, stéarates, linoléates, linolénates, caprates, arachidonates, ou leurs mélanges comme notamment les esters mixtes oléo-palmitates, oléo-stéarates, palmito-stéarates.

Plus particulièrement, on préfère utiliser les mono- et di- esters et notamment les mono- ou di- oléates, stéarates, béhénates, oléopalmitates, linoléates, linolénates, oléostéarates, de saccharose, de glucose ou de méthylglucose.

Les huiles de silicone peuvent aussi être employées comme liquide organique inerte.

Plus particulièrement, les huiles de silicone convenables sont des fluides de silicones liquides et non volatiles de viscosité inférieure ou égale à 10 000 mPa.s à 25 °C, la viscosité des silicones étant mesurée selon la norme ASTM 445 Appendice C.

Les huiles de silicone sont définies plus en détail dans l'ouvrage de Walter NOLL "Chemistry and Technology of Silicones" (1968) - Academic Press.

Parmi les huiles de silicone utilisables selon l'invention, on peut citer notamment les huiles de silicones vendues sous les dénominations DC-200 fluid - 5 mPa.s, DC-200 fluid - 20 mPa.s, DC-200 fluid - 350 mPa.s, DC-200 fluid - 1000 mPa.s, DC-200 fluid - 10 000 mPa.s par la société Dow Corning.

Les huiles minérales peuvent aussi être utilisées comme liquide inerte organique, comme par exemple l'huile de paraffine.

Les huiles végétales peuvent aussi convenir, et notamment l'huile d'avocat, l'huile d'olive ou la cire liquide de jojoba.

De préférence, le ou les liquides inertes organiques additionnels sont choisis dans le groupe formé par les polydécènes de formule C₁₀ₙH_{[(20n)+2]} dans laquelle n varie de 3 à 9 et de préférence de 3 à 7, les esters d'alcools gras ou d'acides gras différents des esters de l'invention, et leurs mélanges.

La teneur en liquides inertes organiques additionnels peut varier entre 5 et 60 %, de préférence entre 10 et 50 % en poids, et encore plus préférentiellement entre 15 et 45 % en poids du poids total de la composition.

Selon un mode de réalisation particulier, la composition conforme à l'invention se présente sous la forme d'une pâte anhydre.

Dans le cadre de la présente invention, une composition est anhydre lorsqu'elle présente une teneur en eau inférieure à 1 % en poids, et de préférence inférieure à 0,5 % en poids par rapport au poids total de la composition.

Selon un autre mode de réalisation particulier, la composition conforme à l'invention comprend de plus du peroxyde d'hydrogène.

Dans ce cas-là, la composition conforme à l'invention est prête à l'emploi et résulte du mélange d'une composition se présentant sous la forme d'une pâte anhydre conforme à l'invention avec une composition aqueuse comprenant du peroxyde d'hydrogène. Son pH est généralement compris entre les valeurs 3 et 11. Il est de préférence compris entre 7 et 11.

La composition conforme à la présente invention peut également comprendre divers additifs classiquement utilisés en cosmétique.

La composition conforme à la présente invention peut ainsi comprendre des agents épaississants minéraux ou organiques, et en particulier des polymères épaississants associatifs ou non, anioniques, cationiques, non ioniques ou amphotères, des charges telles que des argiles, des liants tels que la vinylpyrrolidone, des lubrifiants comme les stéarates de polyol ou les stéarates de métaux alcalins ou alcalino-terreux, des silices hydrophiles ou hydrophobes, des pigments, des colorants, des agents matifiants comme les oxydes de titane ou encore des agents tensioactifs anioniques, non ioniques, cationiques, amphotères ou zwittérioniques, des agents antioxydants, des agents de pénétration, des agents séquestrants, des tampons, des agents dispersants, des agents filmogènes, des agents conservateurs, des agents opacifiants, des vitamines, des parfums, des polymères anioniques, cationiques, non ioniques, amphotères ou zwittérioniques, des céramides, des agents de conditionnement tels que par exemple des silicones volatiles ou non volatiles, modifiées ou non modifiées.

Dans le cas où la composition conforme à l'invention comprend du peroxyde d'hydrogène, elle peut également comprendre des agents de contrôle du dégagement d'oxygène tels que le carbonate ou l'oxyde de magnésium.

Les additifs et les agents de contrôle du dégagement d'oxygène tels que définis précédemment peuvent être présents en quantité comprise pour chacun d'eux entre 0,01 et 40 % en poids, de préférence entre 0,1 et 30 % en poids par rapport au poids total de la composition.

Bien entendu, l'homme de l'art veillera à choisir ce ou ces éventuels composés complémentaires de manière telle que les propriétés avantageuses attachées intrinsèquement à la composition conforme à l'invention ne soient pas, ou substantiellement pas, altérées par la ou les adjonctions envisagées.

Le procédé de décoloration conforme à la présente invention consiste à appliquer sur les fibres kératiniques une composition se présentant sous la forme d'une pâte anhydre conforme à l'invention telle que définie précédemment en présence d'une composition aqueuse comprenant du peroxyde d'hydrogène.

La composition aqueuse comprenant du peroxyde d'hydrogène peut être ajoutée à la composition se présentant sous la forme d'une pâte anhydre juste au moment de l'emploi. Elle peut aussi être appliquée simultanément ou séquentiellement à la composition se présentant sous la forme d'une pâte anhydre.

La présente invention a également pour objet un dispositif à plusieurs compartiments comprenant au moins deux compositions conditionnées séparément dont le mélange conduit à une composition comprenant du peroxyde d'hydrogène conforme à l'invention telle que définie précédemment.

Selon un mode de réalisation particulier, le dispositif conforme à invention comporte un premier compartiment qui contient une composition sous la forme d'une pâte anhydre conforme à la présente invention telle que définie précédemment, et un deuxième compartiment qui contient une composition aqueuse comprenant du peroxyde d'hydrogène.

Le milieu cosmétique approprié de la composition aqueuse comprenant du peroxyde d'hydrogène est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; le glycérol ; les glycols et éthers de glycols comme le 2-butoxyéthanol, le propylèneglycol, le monométhyléther de propylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids par rapport au poids total de la composition tinctoriale, et plus préférentiellement encore entre 5 et 30 % en poids environ.

La composition aqueuse comprenant du peroxyde d'hydrogène présente de préférence un pH inférieur à 7, le pH acide garantissant la stabilité du peroxyde d'hydrogène dans cette composition.

La composition aqueuse comprenant du peroxyde d'hydrogène peut se présenter sous des formes diverses, telles que sous forme de liquides, de crèmes, de gels, ou sous toute autre forme appropriée pour réaliser une décoloration des fibres kératiniques.

La composition aqueuse comprenant du peroxyde d'hydrogène peut également renfermer divers additifs classiquement utilisés en cosmétique tels que ceux qui sont décrits précédemment.

La composition aqueuse comprenant du peroxyde d'hydrogène peut de plus comprendre des agents de contrôle du dégagement d'oxygène tels que définis précédemment.

Le dispositif conforme à la présente invention peut être équipé d'un moyen permettant de délivrer sur les cheveux le mélange souhaité, tel que les dispositifs décrits dans le brevet FR-2 586 913 au nom de la demanderesse.

A partir de ce dispositif, il est possible de décolorer les fibres kératiniques à partir d'un procédé conforme à l'invention tel que défini précédemment.

La présente invention a aussi pour objet l'utilisation d'au moins un ester liquide ramifié non volatile d'acide carboxylique dont le point de solidification est inférieur à 4 °C tel que défini précédemment dans une composition pour la décoloration des fibres kératiniques sous la forme d'une pâte anhydre comprenant au moins un sel peroxygéné.

Selon un mode de réalisation particulier, l'utilisation conforme à l'invention permet d'améliorer la résistance de la composition pour la décoloration des fibres kératiniques aux basses températures, et en particulier permet d'éviter le problème de synérèse lors de stockage à basses températures et lors de transport incluant des cycles de température.

L'invention va être plus complètement illustrée à l'aide des exemples non limitatifs suivants.

Les pâtes anhydres de décoloration suivantes ont été préparées :

| **Composition** | **A** | **B** |
|---|---|---|
| | **(invention)** | **(art antérieur)** |
| Persulfate de sodium | 5,91 g | 5,91 g |
| Disilicate de sodium hydrate | 12,78 g | 12,78 g |
| Persulfate de potassium | 36 g | 36 g |
| Acide éthylènediamine tétraacétique | 0,17 g | 0,17 g |
| Silice pyrogénée à caractère hydrophile | 1,75 g | 1,75 g |
| Oxyde de titane | 0,34 g | 0,34 g |
| Myristate d'isopropyle | 0,64 g | 33,78 g |
| Cire d'abeille blanche | 0,1 g | 0,1 g |
| Isononanoate d'isononyle | 33,14 g | - |
| N-oleyl di-hydrosphingosine | 0,01 g | 0,01 g |
| Gomme de guar | 0,85 g | 0,85 g |
| Hydroxyéthyl cellulose | 0,64 g | 0,64 g |
| Carboxyméthyl amidon de pomme de terre | 2,56 g | 2,56 g |
| Lauryl sulfate de sodium | 3,41 g | 3,41 g |
| Stéarate de magnésium | 1,7 g | 1,7 g |

Chacune des pâtes décolorantes décrites ci-dessus a été soumise à différents tests afin d'évaluer leur résistance au froid et au transport.

### Test 1

Chacune des pâtes décolorantes décrites ci-dessus a été placée pendant une semaine au réfrigérateur à 4 °C. Après retour à la température ambiante, on a observé ce qui suit :
- la composition A ne présente aucune modification ;
- dans le cas de la composition B, on observe le durcissement de la pâte puis la formation de poches d'huile.

### Test 2

Chacune des pâtes décolorantes décrites ci-dessus a été soumise à 2 cycles de température de 20 °C à - 20 °C (un cycle = 6 heures à 20 °C, puis de 20 °C à - 20 °C en 6 heures, puis 6 heures à - 20 °C, puis de - 20 °C à 20 °C en 6 heures), suivi d'une agitation de 1 heure simulant les vibrations subies par un échantillon lors d'un transport par camion sur une distance de 1000 km. On a observé ce qui suit :
- la composition A ne présente aucune modification;
- dans le cas de la composition B, un déphasage d'huile en surface de la pâte est observé.

### Test 3

Pour chacune des pâtes décolorantes décrites ci-dessus, on a enregistré par calorimétrie différentielle à balayage leur comportement thermique lors d'un refroidissement de 25 °C à - 40 °C à la vitesse de 5 °C / minute. On a observé ce qui suit :
- la composition A ne montre aucune transition ;
- la composition B présente un pic prononcé de cristallisation débutant à - 4 °C.

En conclusion, on a observé une supériorité nette de la résistance au froid et au transport de la pâte anhydre comprenant de l'isononanoate d'isononyle par rapport à la pâte anhydre comprenant du myristate d'isopropyle.

## Revendications

1. Composition pour la décoloration des fibres kératiniques, sous la forme d'une pâte anhydre comprenant au moins un sel peroxygéné et au moins un ester liquide ramifié non volatile d'acide carboxylique dont le point de solidification, determiné par DSC, est inférieur à 4 °C choisi parmi les composés de formule (I) suivante :
R₁-CO-O-R₂ (I)
dans laquelle R₁ et R₂ désignent, indépendamment l'un de l'autre, une chaîne hydrocarbonée en C₁-C₃₀ éventuellement interrompue par un ou plusieurs atomes d'oxygène et / ou par un ou plusieurs groupements carbonyle et éventuellement substituée par un ou plusieurs groupements hydroxy, R₁ étant ramifiée.

2. Composition selon la revendication 1 dans laquelle le ou les sels peroxygénés sont choisis parmi les persulfates, les perborates, les percarbonates, les peroxydes de métaux alcalins ou alcalino-terreux, et leurs mélanges.

3. Composition selon la revendication 2 dans laquelle le ou les sels peroxygénés sont choisis parmi les persulfates et leurs mélanges.

4. Composition selon l'une quelconque des revendications précédentes dans laquelle la concentration en sels peroxygénés est comprise entre 10 et 70 % en poids du poids total de la composition.

5. Composition selon l'une quelconque des revendications précédentes dans laquelle le ou les esters liquides ramifiés non volatiles d'acides carboxyliques dont le point de solidification est inférieur à 4 °C comprennent au moins 8 atomes de carbone.

6. Composition selon l'une quelconque des revendications précédentes dans laquelle R₁ et R₂ sont ramfiées.

7. Composition selon l'une quelconque des revendications précédentes dans laquelle l'ester liquide ramifié non volatile d'acide carboxylique dont le point de solidification est inférieur à 4 °C est l'isononanoate d'isononyle.

8. Composition selon l'une quelconque des revendications précédentes dans laquelle la concentration en esters liquides ramifiés non volatiles d'acides carboxyliques dont le point de solidification est inférieur à 4 °C est comprise entre 1 et 70 % en poids du poids total de la composition.

9. Composition selon l'une quelconque des revendications précédentes comprenant au moins un agent alcalin.

10. Composition selon la revendication 9 dans laquelle le ou les agents alcalins sont choisis parmi l'urée, les sels d'ammonium, les silicates, les phosphates ou les carbonates de métaux alcalins ou alcalino-terreux, et leurs mélanges.

11. Composition selon la revendication 10 dans laquelle le ou les agents alcalins sont choisis parmi le chlorure d'ammonium, les silicates, les carbonates, et leurs mélanges.

12. Composition selon l'une quelconque des revendications 9 à 11 dans laquelle le ou les agents alcalines sont en quantité comprise entre 0,01 et 40 % en poids du poids total de la composition.

13. Composition selon l'une quelconque des revendications précédentes comprenant au moins un liquide inerte organique additionnel différent des esters liquides ramifiés non volatiles d'acides carboxyliques dont le point de solidification est inférieur à 4 °C tels que définis à l'une quelconque des revendications 1 et 5 à 7.

14. Composition selon la revendication 13 dans laquelle le ou les liquides inertes organiques additionnels sont choisis parmi les polydécènes de formule C₁₀ₙH_{[(20n)+2]} dans laquelle n varie de 3 à 9, les esters d'alcools gras ou d'acides gras différents des esters liquides ramifiés non volatiles d'acides carboxyliques dont le point de solidification est inférieur à 4 °C tels que définis à l'une quelconque des revendications 1 et 5 à 7, les esters ou di-esters de sucres et d'acides gras en C₁₂-C₂₄, les éthers cycliques ou les esters cycliques, les huiles de silicone, les huiles minérales ou les huiles végétales, ou leurs mélanges.

15. Composition selon la revendication 13 ou 14 dans laquelle la teneur en liquides inertes organiques additionnels varie entre 5 et 60 % en poids du poids total de la composition.

16. Composition selon l'une quelconque des revendications 1 à 15 comprenant du peroxyde d'hydrogène.

17. Procédé de décoloration consistant à appliquer sur les fibres kératiniques une composition se présentant sous la forme d'une pâte anhydre telle que définie à l'une quelconque des revendications 1 à 15 en présence d'une composition aqueuse comprenant du peroxyde d'hydrogène.

18. Dispositif à plusieurs compartiments comprenant au moins deux compositions conditionnées séparément dont le mélange conduit à une composition comprenant du peroxyde d'hydrogène telle que définie à la revendication 16.

19. Dispositif selon la revendication 18 comportant un premier compartiment qui contient une composition sous la forme d'une pâte anhydre telle que définie à l'une quelconque des revendications 1 à 15, et un deuxième compartiment qui contient une composition aqueuse comprenant du peroxyde d'hydrogène.

20. Utilisation d'au moins un ester liquide ramifié non volatile d'acide carboxylique dont le point de solidification est inférieur à 4 °C tel que défini à l'une quelconque des revendicatons 1 et 5 à 7 dans une composition pour la décoloration des fibres kératiniques sous la forme d'une pâte anhydre comprenant au moins un sel peroxygéné.

21. Utilisation selon la revendication 20 pour améliorer la résistance de la composition pour la décoloration des fibres kératiniques aux basses températures.

## Patentansprüche

1. Zusammensetzung zum Bleichen von Keratinfasern in Form einer wasserfreien Paste, umfassend mindestens ein Peroxysalz und mindestens einen flüssigen, nichtflüchtigen, verzweigten Carbonsäureester, dessen Erstarrungspunkt, bestimmt mittels DSC, unter 4 °C liegt, ausgewählt aus den Verbindungen mit der folgenden Formel (I):
R₁-CO-O-R₂ (I)
wobei R₁ und R₂ unabhängig voneinander für eine C₁-C₃₀-Kohlenwasserstoffkette stehen, die gegebenenfalls von einem oder mehreren Sauerstoffatomen und/oder von einer oder mehreren Carbonylgruppen unterbrochen und gegebenenfalls mit einem oder mehreren Hydroxygruppen substituiert ist, wobei R₁ verzweigt ist.

2. Zusammensetzung nach Anspruch 1, wobei das oder die Peroxysalze aus den Persulfaten, Perboraten, Percarbonaten, Peroxiden von Alkalimetallen und Erdalkalimetallen oder deren Gemischen ausgewählt sind.

3. Zusammensetzung nach Anspruch 2, wobei das oder die Peroxysalze aus den Persulfaten und deren Gemischen ausgewählt sind.

4. Zusammensetzung nach einem der vorhergehenden Ansprüche, wobei die Konzentration an Peroxysalzen im Bereich zwischen 10 und 70 Gew.-% des Gesamtgewichts der Zusammensetzung liegt.

5. Zusammensetzung nach einem der vorhergehenden Ansprüche, wobei der oder die flüssigen, nichtflüchtigen, verzweigten Carbonsäureester, deren Erstarrungspunkt unter 4 °C liegt, mindestens 8 Kohlenstoffatome umfassen.

6. Zusammensetzung nach einem der vorhergehenden Ansprüche, wobei R₁ und R₂ verzweigt sind.

7. Zusammensetzung nach einem der vorhergehenden Ansprüche, wobei der flüssige, verzweigte, nichtflüchtige Carbonsäureester, dessen Erstarrungspunkt unter 4 °C liegt, Isononylisononanoat ist.

8. Zusammensetzung nach einem der vorhergehenden Ansprüche, wobei die Konzentration an flüssigen, verzweigten, nichtflüchtigen Carbonsäureestern, deren Erstarrungspunkt unter 4 °C liegt, im Bereich zwischen 1 und 70 Gew.-% des Gesamtgewichts der Zusammensetzung liegt.

9. Zusammensetzung nach einem der vorhergehenden Ansprüche, umfassend mindestens ein alkalisches Mittel.

10. Zusammensetzung nach Anspruch 9, wobei das oder die alkalischen Mittel aus Harnstoff, den Ammoniumsalzen, Silicaten, Phosphaten oder Carbonaten von Alkalimetallen und Erdalkalimetallen oder deren Gemischen ausgewählt sind.

11. Zusammensetzung nach Anspruch 10, wobei das oder die alkalischen Mittel aus Ammoniumchlorid, den Silicaten, den Carbonaten und deren Gemischen ausgewählt sind.

12. Zusammensetzung nach einem der Ansprüche 9 bis 11, wobei das oder die alkalischen Mittel in einer Menge im Bereich zwischen 0,01 und 40 Gew.-% des Gesamtgewichts der Zusammensetzung vorhanden sind.

13. Zusammensetzung nach einem der vorhergehenden Ansprüche, umfassend mindestens eine zusätzliche inerte, organische Flüssigkeit, die von den flüssigen, verzweigten, nichtflüchtigen Carbonsäureestern, deren Erstarrungspunkt unter 4 °C liegt, wie in einem der Ansprüche 1 und 5 bis 7 definiert, verschieden ist.

14. Zusammensetzung nach Anspruch 13, wobei die zusätzliche(n) inerte(n), organische(n) Flüssigkeit(en) ausgewählt sind aus den Polydecenen mit der Formel C₁₀ₙH_{[(20n)+2]}, worin n von 3 bis 9 variiert, den Fettalkoholestern oder Fettsäureestern, die von den flüssigen, verzweigten, nichtflüchtigen Carbonsäureestern, deren Erstarrungspunkt unter 4 °C liegt, wie in einem der Ansprüche 1 und 5 bis 7 definiert, verschieden sind, den Estern oder Diestern von Zuckern und C₁₂-C₂₄-Fettsäuren, den zyklischen Ethern oder den zyklischen Estern, den Siliconölen, den Mineralölen oder den Pflanzenölen oder deren Gemischen.

15. Zusammensetzung nach Anspruch 13 oder 14, wobei der Gehalt an zusätzlichen inerten organischen Flüssigkeiten zwischen 5 und 60 Gew.-% des Gesamtgewichts der Zusammensetzung variiert.

16. Zusammensetzung nach einem der Ansprüche 1 bis 15, umfassend Wasserstoffperoxid.

17. Verfahren zum Bleichen, das darin besteht, auf den Keratinfasern eine Zusammensetzung, die die Form einer wasserfreien Paste aufweist, wie in einem der Ansprüche 1 bis 15 definiert, in Gegenwart einer wässrigen Zusammensetzung, die Wasserstoffperoxid umfasst, anzuwenden.

18. Vorrichtung mit mehreren Kammern, umfassend mindestens zwei Zusammensetzungen, die getrennt verpackt sind, deren Mischung zu einer Zusammensetzung führt, die Wasserstoffperoxid umfasst, wie in Anspruch 16 definiert.

19. Vorrichtung nach Anspruch 18, umfassend eine erste Kammer, die eine Zusammensetzung in Form einer wasserfreien Paste, wie in einem der Ansprüche 1 bis 15 definiert, enthält, und eine zweite Kammer, die eine wässrige Zusammensetzung enthält, die Wasserstoffperoxid umfasst.

20. Verwendung mindestens eines flüssigen, verzweigten, nichtflüchtigen Carbonsäureesters, dessen Erstarrungspunkt unter 4 °C liegt, wie in einem der Ansprüche 1 und 5 bis 7 definiert, in einer Zusammensetzung zum Bleichen von Keratinfasern in Form einer wasserfreien Paste, umfassend mindestens ein Peroxysalz.

21. Verwendung nach Anspruch 20, um die Beständigkeit der Zusammensetzung zum Bleichen der Keratinfasern bei niedrigen Temperaturen zu verbessern.

## Claims

1. Composition for bleaching keratin fibres, in the form of an anhydrous paste, comprising at least one peroxygenated salt and at least one non-volatile liquid branched ester of a carboxylic acid, the solidification point of which, determined by DSC, is below 4°C, chosen from the compounds of following formula (I):
R₁-CO-O-R₂ (I)
in which R₁ and R₂ denote, independently of one another, a C₁-C₃₀ hydrocarbon-based chain optionally interrupted with one or more oxygen atoms and/or with one or more carbonyl groups and optionally substituted with one or more hydroxyl groups, R₁ being branched.

2. Composition according to Claim 1, in which the peroxygenated salt(s) is (are) chosen from persulfates, perborates, percarbonates and peroxides of alkali metals or alkaline earth metals, and mixtures thereof.

3. Composition according to Claim 2, in which the peroxygenated salt(s) is (are) chosen from persulfates and mixtures thereof.

4. Composition according to any one of the preceding claims, in which the concentration of peroxygenated salts is between 10% and 70% by weight of the total weight of the composition.

5. Composition according to any one of the preceding claims, in which the non-volatile liquid branched ester(s) of carboxylic acids, the solidification point of which is below 4°C, comprise(s) at least 8 carbon atoms.

6. Composition according to any one of the preceding claims, in which R₁ and R₂ are branched.

7. Composition according to any one of the preceding claims, in which the non-volatile liquid branched ester of a carboxylic acid, the solidification point of which is below 4°C, is isononyl isononanoate.

8. Composition according to any one of the preceding claims, in which the concentration of non-volatile liquid branched esters of carboxylic acids, the solidification point of which is below 4°C, is between 1% and 70% by weight of the total weight of the composition.

9. Composition according to any one of the preceding claims, comprising at least one alkaline agent.

10. Composition according to Claim 9, in which the alkaline agent(s) is (are) chosen from urea, ammonium salts, silicates, phosphates or carbonates of alkali metals or alkaline earth metals, and mixtures thereof.

11. Composition according to Claim 10, in which the alkaline agent(s) is (are) chosen from ammonium chloride, silicates, carbonates, and mixtures thereof.

12. Composition according to any one of Claims 9 to 11, in which the alkaline agent (s) is (are) in an amount of between 0.01% and 40% by weight of the total weight of the composition.

13. Composition according to any one of the preceding claims, comprising at least one additional inert organic liquid other than the non-volatile liquid branched esters of carboxylic acids, the solidification point of which is below 4°C, as defined in any one of Claims 1 and 5 to 7.

14. Composition according to Claim 13, in which the additional inert organic liquid(s) is (are) chosen from polydecenes of formula C₁₀ₙH_{[(20n)+2]} in which n ranges from 3 to 9, esters of fatty alcohols or of fatty acids other than the non-volatile liquid branched esters of carboxylic acids, the solidification point of which is below 4°C, as defined in any one of Claims 1 and 5 to 7, esters or diesters of sugars and of C₁₂-C₂₄ fatty acids, cyclic ethers or cyclic esters, silicone oils, mineral oils or vegetable oils, or mixtures thereof.

15. Composition according to Claim 13 or 14, in which the content of additional inert organic liquids ranges between 5% and 60% by weight of the total weight of the composition.

16. Composition according to any one of Claims 1 to 15, comprising hydrogen peroxide.

17. Bleaching process, consisting in applying, to keratin fibres, a composition which is in the form of an anhydrous paste as defined in any one of Claims 1 to 15 in the presence of an aqueous composition comprising hydrogen peroxide.

18. Multicompartment device comprising at least two compositions packaged separately, the mixing of which results in a composition comprising hydrogen peroxide as defined in Claim 16.

19. Device according to Claim 18, comprising a first compartment which contains a composition in the form of an anhydrous paste as defined in any one of Claims 1 to 15, and a second compartment which contains an aqueous composition comprising hydrogen peroxide.

20. Use of at least one non-volatile liquid branched ester of a carboxylic acid, the solidification point of which is below 4°C, as defined in any one of Claims 1 and 5 to 7, in a composition for bleaching keratin fibres, in the form of an anhydrous paste, comprising at least one peroxygenated salt.

21. Use according to Claim 20, for improving the resistance to low temperatures of the composition for bleaching keratin fibres.
